# EUROPÄISCHE PATENTSCHRIFT

(11) **EP 0 615 450 B1**
(45) Veröffentlichungstag und Bekanntmachung des Hinweises auf die Patenterteilung: **22.01.1997**
(21) Anmeldenummer: 92918592.4
(22) Anmeldetag: 02.09.1992
(51) Int. Cl.: A61K 31/80, A61K 9/00, A61K 9/20

(54) **DIMETICON-PASTILLEN**
DIMETICON PASTILLES
PASTILLES DE DIMETICON

(30) Priorität: 05.12.1991 DE 4140116
(43) Veröffentlichungstag der Anmeldung: 21.09.1994
(73) Patentinhaber: BOLDER ARZNEIMITTEL GmbH, D-50968 Köln (DE)
(72) Erfinder: IMER, Faruk, D-5000 Köln 60 (DE)
(74) Vertreter: Jönsson, Hans-Peter, Dr.Dipl.-Chem.
(86) Internationale Anmeldenummer: EP9202014
(87) Internationale Veröffentlichungsnummer: WO9310797

(56) Entgegenhaltungen:
- GB-A- 2 033 915
- US-A- 2 951 011
- US-A- 3 382 150
- Pharmazeutische Zeitung, aus 127. Jahrgang, Nr. 41, Seiten 2214-2218, Oktober 1982; W. RAHN: "Arzneiformen zum Lutschen"

## Beschreibung

Die Erfindung betrifft Lutsch-Pastillen auf der Basis bestimmter natürlicher oder synthetischer Polymere, enthaltend Poly-(dimethylsiloxane) als Wirkstoff, der unter dem internationalen Freinamen Dimeticon oder Simethicone im Handel erhältlich ist.

Unter Pastillen - vgl. auch W. Rahn, Pharmazeutische Zeitung, S. 2214-2218 (1982) - versteht man im allgemeinen Zubereitungen, die im Munde gelutscht oder zerkaut werden. Und zwar unterscheidet man im wesentlichen zwischen Tabletten, Hartbonbons und Gummipastillen (auch genannt Gummibonbons).

Die Verfahren zur Herstellung dieser Darreichungsformen unterscheiden sich grundsätzlich voneinander.

Tabletten werden auf Tablettenmaschinen gepreßt. Die Tablettenmasse muß dazu durch Mischen und Granulieren vorbereitet werden. Verschiedene Autoren haben sich bereits mit Granulierungsmethoden des schwierig zu verarbeitenden Dimeticons beschäftigt.

Bonbons werden in der Weise hergestellt, daß man Saccharose und Glukosesirup mischt, bei ca. 130° C kocht und der Masse im Vakuum das Wasser bis auf 0,5 bis 2 % entzieht. Der auf ca. 85° C abgekühlten zähen Bonbonmasse werden die Wirk- und Aromastoffe zugesetzt und diese durch Kneten untergemischt. In Kegelrollern und anderen Bonbonmaschinen wird die Bonbonmasse unter stetigem Abkühlen zu Strängen ausgezogen, geformt und geschnitten. Bekanntermaßen ist die Wirkstoffverteilung, bedingt durch die Zähigkeit der Bonbonmasse, ziemlich ungenau.

Gummipastillen werden in der Weise hergestellt, daß man zunächst in einem Rührwerkskessel Hydrokolloide, wie z. B. Gummi arabicum, zusammen mit Saccharose, Glukosesirup, Sorbitol, Xylitol o. ä. in Wasser auflöst und in dieser Grundmasse die Wirkstoffe emulgiert bzw. suspendiert. Die so erhaltene Gießmasse wird in sogenannten Puderhorden ausgegossen. Dies sind beispielsweise flache Holzkisten von ca. 80 x 40 cm, die mit Stärke, insbesondere Maisstärke gefüllt sind. In dem glattgestrichenen Puder werden mittels eines Stempelbretts die gewünschten Formen eingedrückt und in die erhaltenen Vertiefungen die warme Gießmasse genau dosiert, eingepumpt, wobei sich die eingegossene Masse nicht mit dem Puder verbindet. Horde um Horde, je 500 bis 1000 Pastillen, wird so gegossen, gestapelt und in Trockenkammern im Verlauf von 3 bis 4 Tagen den Pastillen das Wasser bis auf etwa 10 % Restfeuchte entzogen. Die so gefertigten Pastillen werden "ausgepudert" und dann einer Endbehandlung zugeführt.

Dimeticon ist ein nicht wasserlösliches Polysiloxan mit einem Molekulargewicht von etwa 1000 bis 3000, das seit langem als Antiflatulenzmittel von verschiedenen Anbietern in verschiedenen Darreichungsformen vertrieben wird. Beispielsweise sind Tabletten, Kapseln, Kautabletten und Tropfen im Handel erhältlich. Bedingt durch die hochviskose ölige Konsistenz von Dimeticon läßt sich die Substanz ohne Hilfs- oder Trägerstoffe nicht direkt in Tablettenform pressen. Dementsprechend wird häufig im Stand der Technik Dimeticon mit hochdispersen Trägern wie Siliciumdioxid oder Aluminiumoxid vermengt und so, gegebenenfalls unter Verwendung von Tablettierhilfsstoffen oder Tablettierhilfsmitteln in Tabletten eingearbeitet. Auch bei der Herstellung von Tropfen wird vielfach hochdisperses Siliciumdioxid eingesetzt.

Bei der oralen Applikation von Tabletten, Kapseln oder auch Tropfen wird der Wirkstoff Dimeticon im Magen-Darmtrakt freigesetzt und entfaltet seine Wirkung durch Herabsetzung der Oberflächenspannung. Bedingt durch die hohe Viskosität, ölige Beschaffenheit und stark hydrophobe Eigenschaft des Dimeticons ist jedoch vielfach eine feine Dispersion des Wirkstoffes nicht zu erreichen, so daß insbesondere bei Kautabletten eine mehr oder weniger große Granulatbildung auftritt. Nachteilig bei der Darreichung in Tropfenform ist, daß die gesamte Wirkstoffmenge in einem relativ kurzen Zeitraum abgegeben wird und nur unverteilt zur Verfügung steht.

Aus Derwent Abstracts 91-60 235 (HU-00 5450) sind oral zu verabreichende Zusammensetzungen zur Behandlung der Flatulenz bekannt, in denen Dimeticon mit Trägern auf der Basis wasserlöslicher Trägermaterialien, die im Bereich von 40 bis 120° C unzersetzt schmelzen. 10 bis 25 Gew.-% einer vorzugsweise geschmolzenen Mischung werden zu dem geschmolzenen Träger gegeben. Die Mischung enthält natürliche und synthetische Körper, Vernetzungs- und Verdickungsmittel, insbesondere Polyvenylchlorid, Polyvinylacetat, Dextran, mikrokristalline Cellulose, Polyacrylsäure, Polyvinylpyrrolidon und/oder anorganische Stabilisatoren. Eine Darreichungsform wird nicht erwähnt. Vielmehr wird ein spezielles Beschichtungsverfahren zur Herstellung von Dimeticontabletten beschrieben.

Aus EP-A-0 425 450 sind Antiflatulenzmittel bekannt, die aus einer freifließenden granularen Mischung aus 50 bis 90 Gew.-% eines wasserlöslichen agglomerierten Maltodextrins und 10 bis 50 Gew.-% eines flüssigen, nicht wäßrigen Silikonöls, beispielsweise Dimeticon, bestehen. Die Zusammensetzung wird mit weiteren Hilfsstoffen vermischt und in einzel dosierter Form zu Tabletten, gefüllten Kapseln oder Granulaten verarbeitet.

Aus US-A-4 396 604 sind Pastillen bekannt, die neben einem Antacidum Dimeticon enthalten. Die hier beschriebenen Hartbonbons werden dadurch hergestellt, daß man eine Lösung oder Suspension von Zucker oder Zuckeraustauschstoffen mit einem Antacidum bei hoher Temperatur versetzt, und unter Vakuum das Wasser weitgehend verdampft. Nach Abkühlen der Mischung, beispielsweise auf 85° C wird dann unter Kneten das Dimeticon zugegeben. Die Bonbonmasse wird in Stränge gezogen und in Bonbonmaschinen geschnitten bzw. geformt. Dabei resultieren relativ schwere Bonbons mit einem Gewicht von 3 bis 6 g, jedoch mit ungenauer Wirkstoffverteilung.

Die Aufgabe der vorliegenden Erfindung bestand demgegenüber darin, unter Anwendung eines an sich aus der Süßwarenherstellung bekannten Gieß-Verfahrens, eine neue Darreichungsform für Dimeticon zur Verfügung zu stellen, die sich auszeichnet durch: feinste Verteilung des Wirkstoffes in der Pastille, sehr genaue Einzeldosierung des Wirkstoffes, einfache Handhabung eines sonst schwer zu handhabenden Wirkstoffes, angenehme Einnahme des Arzneimittels und optimale Verteilung des Wirkstoffes im Magen durch das langsame Lutschen der Pastille.

Gelöst wird die vorgenannte Aufgabe durch Pastillen auf der Basis von wenigstens teilweise oder vollständig wasserlöslichen, natürlichen und/oder synthetischen Polymeren, ausgewählt aus Gummen, Alginaten, Carragen, Stärke, Pektin und Gelatine, die in wäßrigen Systemen Gele oder viskose Lösungen bilden und weiteren Hilfs- und Zusatzstoffen, enthaltend Poly-(dimethylsiloxane) (Dimeticon). Überraschenderweise wurde gefunden, daß das hochviskose, nicht wasserlösliche und stark hydrophobe Dimeticon sich in außerordentlich einfacher Weise feindispers in Pastillen auf der Basis der genannten natürlichen und/oder synthetischen Polymere einarbeiten läßt.

Der Begriff Pastillen und insbesondere der Begriff Gummipastillen beinhaltet im Sinne der vorliegenden Erfindung solche, die durch Gießen hergestellt werden. Dementsprechend bestehen die Pastillen gemäß der vorliegenden Erfindung aus verschiedenen geformten elastischen Formkörpern, die in einer Mischung von Hydrokolloiden und weiteren Hilfs- und Zusatzstoffen Dimeticon in feinster Verteilung enthalten. Gummipastillen werden als feste Lösungen bezeichnet, die beim Lutschen wieder in flüssige Lösungen zurückverwandelt werden. Mit Hilfe der vorliegenden Erfindung ist es möglich, eine genaue Einzeldosierung der Pastille bei relativ schonender Verarbeitungsweise der Bestandteile zu erreichen. Bei den genannten, wenigstens teilweise oder vollständig wasserlöslichen, natürlichen und/oder synthetischen Polymeren, ist eine besonders gute Einarbeitung des Dimeticons möglich, da hier bei relativ niedrigen Temperaturen gearbeitet werden kann. Somit entsteht eine besonders homogene Verteilung des Wirkstoffes in der Gesamtmasse, die es erlaubt, die Dosierung des Wirkstoffes mit Standardabweichungen im Bereich von 0,5 bis 2 % zu dosieren. Darüberhinaus ist mit Hilfe des erfindungsgemäßen Verfahrens die Herstellung von relativ hochkonzentrierten Dimeticon-Pastillen möglich.

Besonders bevorzugte natürliche und/oder synthetische Polymere im Sinne der vorliegenden Erfindung sind auch unter dem Begriff "Hydrokolloide" bekannt. Besonders bevorzugt sind die Gummen, ausgewählt aus Gummi arabicum, Gelatine oder Traganth. In gleicher Weise sind die Hilfs- und Zusatzstoffe vorzugsweise ausgewählt aus Zucker und/oder Zuckeraustauschstoffen, hydrierten Fetten, Stearinsäure, Paraffinen, Oligosacchariden, Polysacchariden und/oder Dextran. Besonders bevorzugte Saccharide sind Saccharose und/oder Glukosesirup.

Entsprechende Pastillen mit anderen Wirkstoffen sind an sich im Stand der Technik unter dem Begriff "Gummipastillen" bekannt. Diese leiten ihren Namen von dem in ihnen verarbeiteten Rohstoff Gummi arabicum ab. Auch im Sinne der vorliegenden Erfindung wird dieses Hydrokolloid als Grundstoff besonders bevorzugt, weil es beim Lutschen der Pastille ein langsames und gleichmäßiges Abschmelzen des Pastillenkörpers gewährleistet.

Neben den Polymeren enthält die Grundmasse insbesondere Geschmacksträger wie Zucker und/ oder Zuckeraustauschstoffe, da der Patient die Pastille lutschen soll. Dementsprechend ist es erforderlich, daß die Pastillen so gut schmecken, daß diese nicht verweigert oder aber hinuntergeschluckt werden. Zur Geschmacksverbesserung werden, wie an sich im Stand der Technik bekannt, entsprechende Hilfsstoffe wie Saccharose oder deren Austauschstoffe Fruktose, hydrierter Glukosesirup, Sorbit, Mannit und/oder Xylit sowie bekannte Süßstoffe eingesetzt. Daneben können auch Geschmackskorrigenzien und Essenzen, ebenso wie etherische Öle, eingesetzt werden und vereinen damit therapeutische Wirkung und Geschmacksverbesserung. Insbesondere etherische Öle, wie Kümmelöl oder Fenchelöl, sind geeignet, die Antiflatulenzwirkung des Dimethicons zu verstärken.

Der Nachteil von Kautabletten besteht unter anderem darin, daß die Tabletten durch das Kauen in viele unterschiedlich große Tablettenstücke oder Granulatkörner zerteilt werden, die in dieser Form in den Magen gelangen, sich dort in der Regel aber nicht weiter auflösen, so daß nur ein begrenzter Teil des Wirkstoffes zur Verfüung steht.

Die Pastillen gemäß der vorliegenden Erfindung werden dagegen durch den Speichel beim Lutschen in eine Emulsion oder Suspension zurückverwandelt, in der der Wirkstoff in feinstverteilter Form vorliegt und somit vollständig über einen längeren Zeitraum zur Verfügung steht.

Die Mengenanteile der jeweils notwendigen Bestandteile der Pastillen sind weniger kritisch. Dementsprechend enthält die Grundmasse beispielsweise 5 bis 70 Gew.% der Polymere, bezogen auf die Gesamtmasse der Pastillen. Besonders bevorzugt ist eine Menge von 25 bis 60 Gew.% Gummi arabicum oder 5 bis 20 Gew.% Gelatine, jeweils bezogen auf die Gesamtmasse der Pastillen.

In einer weiteren bevorzugten Ausführungsform der vorliegenden Erfindung enthalten Pastillen 20 bis 50 Gew.% Zucker und/oder Zuckeraustauschstoffe bezogen auf die Gesamtmasse der Pastillen.

Im Handel erhältliche Präparate wie Kautabletten, Kapseln oder Tropfen mit dem Wirkstoff Dimeticon enthalten üblicherweise 10 bis 200 mg Dimeticon pro Anwendungsdosis. Dementsprechend besteht eine besonders bevorzugte Ausführungsform der vorliegenden Erfindung darin, Pastillen mit einer Menge von 70 bis 100 mg Dimeticon zur Verfügung zu stellen. Üblicherweise beträgt das Gewicht derart hergestellter Pastillen etwa 1 g.

Eine weitere Ausführungsform der vorliegenden Erfindung besteht in dem Verfahren zur Herstellung der Pastillen. Hierbei werden insbesondere die oben definierten Polymere mit Wasser und weiteren Hilfs- und Zusatzstoffen unter Bildung eines Gels oder einer viskosen Lösung in Kontakt gebracht. Anschließend wird Dimeticon in der so erhaltenen Grundmasse suspendiert oder emulgiert und dann diese flüssige Masse in Formen gegossen, bei Raumtemperatur oder bei erhöhter Temperatur, insbesondere 40 bis 70° C, bevorzugt 50 bis 60° C, getrocknet, aus der Form entfernt und einer Endbehandlung zugeführt.

Beispielsweise werden zu Beginn des Herstellungsverfahrens Gummi arabicum und Saccharose in Wasser gelöst und in der dickflüssigen Grundmasse Dimeticon emulgiert oder suspendiert. Diese Arzneimittelmischung wird in sogenannten Puderhorden ausgegossen und, wie eingangs beschrieben, getrocknet, vom Puder getrennt und endbehandelt. Die besonderen Vorteile der erfindungsgemäßen Pastillen und des Verfahrens zu ihrer Herstellung bestehen in einer geringen Temperaturbelastung der Hilfs- und Wirkstoffe und deren vollständiger Homogenität in der Gießmasse, die eine hohe Genauigkeit der Wirkstoffdosierung erlaubt. Somit ist der schwierig zu handhabende Wirkstoff Dimeticon in einer neuen Darreichungsform verfügbar.

## Patentansprüche

1. Lutschpastillen auf der Basis von wenigstens teilweise oder vollständig wasserlöslichen natürlichen und/oder synthetischen Polymeren, ausgewählt aus Gummen, Alginaten, Carragen, Stärke, Pektin und Gelatine, die in wäßrigen Systemen Gele oder viskose Lösungen bilden und weiteren Hilfs- und Zusatzstoffen, enthaltend Poly-(dimethylsiloxane) (Dimeticon).

2. Pastillen nach Anspruch 1, dadurch gekennzeichnet, daß die Gummen ausgewählt sind aus Gummi arabicum, Gelatine oder Traganth.

3. Pastillen nach Anspruch 1 oder 2, dadurch gekennzeichnet, daß die Hilfs- und Zusatzstoffe ausgewählt sind aus Zucker und/oder Zuckeraustauschstoffen, hydrierten Fetten, Stearinsäure, Paraffinen, Oligosacchariden, Polysacchariden und/oder Dextran.

4. Pastillen nach einem oder mehreren der Ansprüche 1 bis 3, enthaltend als Zuckeraustauschstoff Fruktose, Sorbit, Mannit, Xylit, hydrierten Glukosesirup und/oder Süßstoffe.

5. Pastillen nach einem oder mehreren der Ansprüche 1 bis 4, enthaltend 5 bis 70 Gew.% Polymere bezogen auf die Gesamtmasse der Pastillen.

6. Pastillen nach einem oder mehreren der Ansprüche 1 bis 5, enthaltend 25 bis 60 Gew.% Gummi arabicum oder 5 bis 20 Gew.% Gelatine, bezogen auf die Gesamtmasse der Pastillen.

7. Pastillen nach einem oder mehreren der Ansprüche 1 bis 6, enthaltend 20 bis 50 Gew.% Zucker und/oder Zuckeraustauschstoffe, bezogen auf die Gesamtmasse der Pastillen.

8. Pastillen nach einem oder mehreren der Ansprüche 1 bis 7, enthaltend 1 bis 20 Gew.-%, insbesondere 7 bis 10 Gew.-% Dimeticon.

9. Verfahren zur Herstellung der Pastillen nach einem oder mehreren der Ansprüche 1 bis 8, wobei man
a) wenigstens teilweise oder vollständig wasserlösliche natürliche und/oder synthetische Polymere, ausgewählt aus Gummen, Alginaten, Carragen, Stärke, Pektin und Gelatine, mit Wasser und weiteren Hilfs- und Zusatzstoffen unter Bildung eines Gels oder einer viskosen Lösung in Kontakt bringt und Dimeticon in der so erhaltenen Grundmasse emulgiert oder suspendiert,
b) die Emulsion gemäß a) in Formen ausgießt, die gegebenenfalls mittels Stempelbrettern in Puderhorden eingedrückt sind und
c) bei Raumtemperatur oder erhöhter Temperatur trocknet.

10. Verfahren nach Anspruch 9, dadurch gekennzeichnet, daß man die Pastillen bei einer Temperatur im Bereich von 40 bis 70° C, insbesondere 50 bis 60° C, trocknet.

11. Verfahren nach Anspruch 9 oder 10, dadurch gekennzeichnet, daß man die getrockneten Pastillen aus der Form entfernt und gegebenenfalls endbehandelt.

## Claims

1. Sucking pastilles based on at least partially or completely water soluble natural and/or synthetic polymers, selected from gums, alginates, carrageen, starch, pectin, and gelatin, which will form gels or viscous solutions in aqueous systems, and additional adjuvants and additives, said pastilles containing poly(dimethylsiloxane) (Dimeticon).

2. The pastilles according to claim 1, characterized in that said gums are selected from gum arabic, gelatin or tragacanth.

3. The pastilles according to claim 1 or 2, characterized in that said adjuvants and additives are selected from sugar and/or sugar substitutes, hydrogenated fats, stearic acid, paraffins, oligosaccharides, polysaccharides, and/or dextran.

4. The pastilles according to one or more of claims 1 to 3, containing fructose, sorbitol, mannitol, xylitol, hydrogenated glucose syrup, and/or artificial sweeteners as said sugar substitutes.

5. The pastilles according to one or more of claims 1 to 4, containing from 5 to 70% by weight of polymers, based on the total mass of the pastilles.

6. The pastilles according to one or more of claims 1 to 5, containing from 25 to 60% by weight of gum arabic or from 5 to 20% by weight of gelatin, based on the total mass of the pastilles.

7. The pastilles according to one or more of claims 1 to 6, containing from 20 to 50% by weight of sugar and/or sugar substitutes, based on the total mass of the pastilles.

8. The pastilles according to one or more of claims 1 to 7, containing from 1 to 20% by weight, particularly from 7 to 10% by weight, of Dimeticon.

9. A process for the preparation of the pastilles according to one or more of claims 1 to 8, wherein
a) at least partially or completely water soluble natural and/or synthetic polymers, selected from gums, alginates, carrageen, starch, pectin, and gelatin, are contacted with water and additional adjuvants and additives to form a gel or a viscous solution, and Dimeticon is emulsified or suspended in the base composition thus obtained;
b) the emulsion according to a) is cast into molds which are optionally stamped into powder trays by means of stamping boards; and
c) dried at room temperature or elevated temperature.

10. The process according to claim 9, characterized in that said pastilles are dried at a temperature in the range of from 40 to 70°C, particularly from 50 to 60°C.

11. The process according to claim 9 or 10, characterized in that the dried pastilles are removed from the mold and optionally subjected to final treatment.

## Revendications

1. Pastilles à sucer à base de polymères naturels et/ou synthétiques au moins partiellement ou complètement solubles dans l'eau sélectionnés parmi les gommes, les alginates, le carraghénane, l'amidon, la pectine et la gélatine, qui forment, dans des systèmes aqueux, des gels ou des solutions visqueuses, et d'autres substances auxiliaires et additives, contenant du poly-(diméthylsiloxane) (Dimeticone).

2. Pastilles selon la revendication 1, caractérisées en ce que les gommes sont sélectionnées parmi la gomme arabique, la gélatine et la gomme adragante.

3. Pastilles selon la revendication 1 ou 2, caractérisées en ce que les substances auxiliaires et additives sont sélectionnées de préférence parmi le sucre et/ou les substituts du sucre, les grasses hydratées, l'acide stéarique, les paraffines, les oligosaccharides, les polysaccharides et/ou le dextranne.

4. Pastilles selon une ou plusieurs des revendications 1 à 3, contenant comme substitut du sucre le fructose, le sorbitol, le mannitol, le xylitol, le sirop de glucose hydraté et/ou des substances édulcorantes.

5. Pastilles selon une ou plusieurs des revendications 1 à 4, contenant 5 % à 70 % de polymères par rapport à la masse totale des pastilles.

6. Pastilles selon une ou plusieurs des revendications 1 à 5, contenant 25 % à 60 % en poids de gomme arabique ou 5 % à 20 % en poids de gélatine par rapport à la masse totale des pastilles.

7. Pastilles selon une ou plusieurs des revendications 1 à 6, contenant 20 % à 50 % en poids de sucre et/ou de substituts du sucre par rapport à la masse totale des pastilles.

8. Pastilles selon une ou plusieurs des revendications 1 à 7, contenant 1 % à 20 % en poids, en particulier 7 % à 10 % en poids de Dimeticone.

9. Procédé de préparation des pastilles selon une ou plusieurs des revendications 1 à 8, dans lequel :
a) on met en contact des polymères naturels et/ou synthétiques au moins partiellement ou complètement solubles dans l'eau, sélectionnées parmi les gommes, les alginates, le carraghénane, l'amidon, la pectine et la gélatine, avec de l'eau et d'autres substances auxiliaires et additives pour former un gel ou une solution visqueuse et on met en émulsion ou en suspension du Dimeticone dans la masse de base ainsi obtenue,
b) on coule l'émulsion selon a) dans des moules qui sont imprimés éventuellement au moyen de planches d'empreinte dans des claies à poudre, et
c) on sèche à température ambiante ou à température élevée.

10. Procédé selon la revendication 9, caractérisé en ce qu'on sèche les pastilles à une température dans la plage de 40°C à 70°C, en particulier de 50°C à 60°C.

11. Procédé selon la revendication 9 ou 10, caractérisé en ce qu'on retire les pastilles séchées du moule et on le soumet éventuellement à un traitement final.
